Europäisches Patentamt

European Patent Office    (11) Publication number:    0 254 767
A1
Office européen des brevets

(19)

# (12) EUROPEAN PATENT APPLICATION

(21) Application number: 86111488.2

(22) Date of filing: 19.08.86

(51) Int. Cl.⁴: **C12P 21/00** , G01N 33/577 , C12N 5/00 , C12N 15/00 , //C07K15/00

(30) Priority: 29.07.86 EP 86110420

(43) Date of publication of application: 03.02.88 Bulletin 88/05

(84) Designated Contracting States: GB

(71) Applicant: **Kishimoto, Tadamitsu, Prof. Division of Cellular Immunology Institute of Molecular and Cellular Biology University Osaka 1-3, Yamadaoka Suita Osaka 565(JP)**

(72) Inventor: **Kishimoto, Tadamitsu, Prof. dr. 3-5-31, Nakaro Tondabayashi Osaka 584(JP)** Inventor: **Suemura, Masaki, Dr. 9-5, Himemuro Ikeda Osaka 563(JP)** Inventor: **Kikutani, Hitoshi, Dr. 2-1-26, Esaka Suita Osaka 564(JP)** Inventor: **Barsumian, Edward L., Dr. 3-1-503, Senba-Nishi Mino Osaka 562(JP)**

(74) Representative: **Laudien, Dieter, Dr. et al Boehringer Ingelheim Zentrale GmbH ZA Patente Postfach 200 D-6507 Ingelheim am Rhein(DE)**

(54) Monoclonal antibodies specific to surface receptor for IgE and hybrid cell lines producing these antibodies and use thereof.

(57) An object of the present application is to provide new anti-Fc$_\epsilon$R-antibodies which enable B cells to be identified at a specific differentiation stage as well enabling the different degrees of atopy and Fc$_\epsilon$R-receptor expression to be defined as a result by the showing the Fc$_\epsilon$R is a B cell specific phenotype marker, the expression of which is strictly correlated with the differentiation stage of B cells, especially with the isotype switching in B cells. Therefore, Fc$_\epsilon$R positive B cells can be recognized by detection with the new antibodies of the expressed Fc$_\epsilon$R, e.g. the expressed Fc$_\epsilon$R in supernatant having a molecular weight of about 25 Kd, and the use thereof in an ELISA.

EP 0 254 767 A1

# Monoclonal antibodies specific to surface receptor for IgE and hybrid cell lines producing these antibodies and use thereof

This invention is concerned with anti-$Fc_\epsilon R$-antibodies specific to surface receptors for IgE, the use thereof and hybrid cell lines producing these antibodies.

It is known from the literature, although the crucial role of IgE is not yet fully appreciated, that IgE biosynthesis requires the interaction of T and B cells and that the regulation of IgE synthesis has certain peculiarities and involves humoral factors derived from other lymphocytes. It is further known that these factors have affinity to IgE and will bind to precursors of IgE-forming cells, thus enhancing or suppressing their differentiation. In this fashion they play a critical role in IgE-isotype-specific regulation.

Furthermore it is known, that B lymphocytes originate from pluripotent hematopoietic stem cells and differentiate into antibody secreting cells through multistep differentiation stages, such as pre-B cells, immature B cells with surface IgM and mature B cells with surface IgM and IgD. A number of human B cell antigens have been defined by monoclonal antibodies (1-10). However, most B cell specific monoclonal antibodies except for antibodies to plasma cells (5) and activated B cells (9,10) are known to recognize cells of the B lineage at a wide range of differentiation stages from pre-B cells to mature B cells. It has therefore been difficult to identify B cells at a specific differentiation stage by employing such B cell-specific monoclonal antibodies.

An object of the present invention is to provide new anti-$Fc_\epsilon R$-antibodies which enable B cells to be identified at a specific differentiation stage as well enabling the different degrees of atopy and $Fc_\epsilon$-receptor expression to be defined as a result by the showing that $Fc_\epsilon R$ is a B cell specific phenotype marker, the expression of which is strictly correlated with the differentiation stage of B cells, especially with the isotype switching in B cells. Therefore, $Fc_\epsilon R$ positive B cells can be recognized by detection with the new antibodies of the expressed $Fc_\epsilon R$, e.g. the expressed $Fc_\epsilon R$ in supernatant having a molecular weight of about 25 Kd.

The new anti-$Fc_\epsilon R$-antibodies were prepared according to the invention as follows:

## Reagents and antibodies:

Human IgE myeloma protein (PS) was the same preparation as described in reference (6). Human $IgG_1$ protein was obtained from Osaka Medical Center and Research Institute for Maternal and Child Health (Dr. A. Shimizu). Fluorescein isothiocyanate (FITC)-conjugated goat anti-mouse IgM + IgG antibodies were purchased from Tago, Inc. Burlingame, Ca.; FITC-conjugated anti-Leu 4 was obtained from Becton-Dickinson Monoclonal Center, Inc. Mountain View, Ca.; FITC-anti BI antibody was obtained from Coulter Immunology, Hialeah, Fl.; the monoclonal anti-B cells antibody, MRII, was prepared as described in reference (12). Avidin-FITC and biotinyl-N-hydroxy succinimide ester were obtained from E.Y. Laboratories, Inc. San Mateo, Ca.; human Igs and monoclonal antibodies were conjugated with biotin or FITC as described in references (13) and (14).

## Human cells lines:

RPMI8866 (8866), an Epstein-Barr virus-transformed B cell line, which is $Fc_\epsilon R$ positive, was obtained from John Hopkins University, Md. (Dr. K. Ishizaka). Other B cell lines (CESS, SKW-CL4 and PRMI1788) and T cell lines (CCRF-CEM and MOLT 4) were obtained from Karolinska Institute, Sweden (Dr. G. Klein). The percentage of $Fc_\epsilon R$-bearing cells by rosetting method was 80-95 % in 8866, CESS and SKW-CL4 cells, 50-65 % in RPMI1788 cells, and 0 % in CEM, MOLT 4 and Daudi cells, respectively.

## Somatic cell hybridization and cloning:

BALB/c mice were immunized i.p. with $1 \times 10^7$ 8866 cells suspended in Hanks' balanced salt solution. Mice were boosted twice with the same number of cells at 4 weeks intervals. Three hundred million spleen cells were fused with $3 \times 10^7$ of hypoxanthine-aminopterine-thymidine (HAT) sensitive P3UI mouse myeloma cells using polyethylene glycol 4000 (Wako Pure Chemical Inc., Osaka, Japan) as described in reference (15). The cells were suspended in HAT medium (Dulbecco's modified Eagle's medium with HAT, 20 % FCS, 1 % NEAA, antibiotics and glutamine) at $1 \times 10^5$ feeder myeloma cells/ml, and 200 $\mu$l of the cell suspension were dispensed with BALB/c thymocytes into 96 well tissue culture plates (Falcon 3072, Oxnard, Ca). One half of the culture medium was changed with fresh HAT medium every third day. After 14 days, the HAT medium was replaced with HT and the hybrid clones were grown in regular culture medium after 3-4 weeks. Wells with growing hybrid cells were screened for anti-Fc$_\epsilon$R antibodies, and positive cultures were cloned by limiting dilutions. After testing for anti-Fc$_\epsilon$R antibody activity, selected monoclonal clones were transplanted in BALB/c mice pretreated with pristane (Aldrich Chemical Co., Milwaukee, Wis.) and ascitic fluid was obtained.

Selection of hybridomas secreting anti-Fc$_\epsilon$R antibodies by immuno-fluorescence:

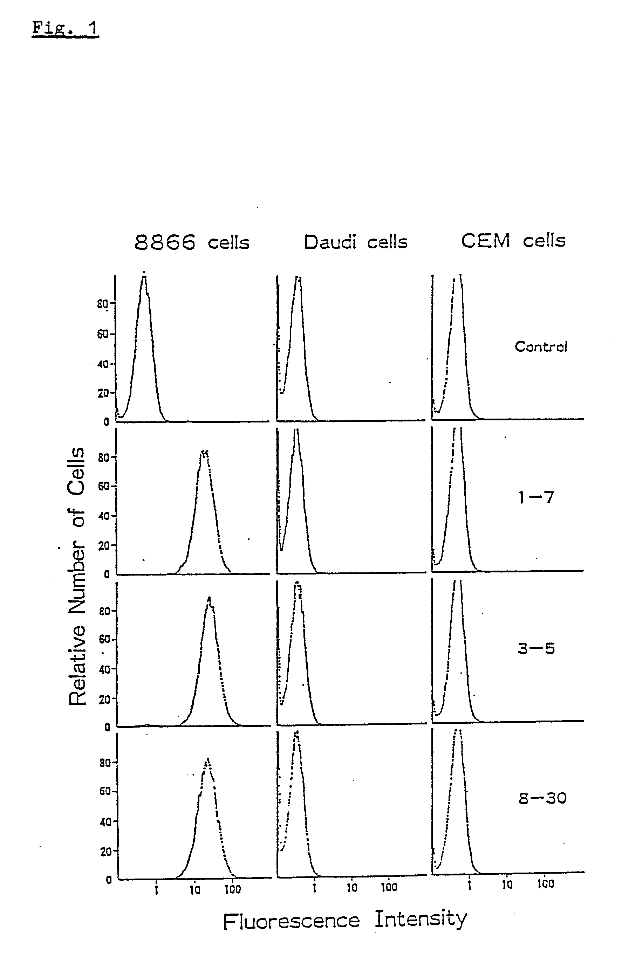

The initial screening of the B hybridomas secreting anti-Fc$_\epsilon$R antibodies was done on the basis of immunofluorescence with 8866, Daudi, CEM, and Molt 4 cells. A half million of 8866, Daudi, DEM and MOLT 4 cells were incubated with 50 $\mu$l of culture supernatants followed by FITC-anti-mouse IgM + IgG and analyzed by a cell sorter (FACS 440, Becton Dickinson, Mountain View, Ca.). After cloning by limiting dilution technique, three hybrid clones, 1-7 ($\gamma$2b), 3-5 ($\gamma$1) and 8-30 ($\mu$), reacting only with 8866 cells but not with Daudi, CEM, and Molt 4 cells, were selected as possible candidates for anti-Fc$_\epsilon$R antibodies (Fig 1.). In order to confirm whether these monoclonal antibodies reacted only with Fc$_\epsilon$R-positive cell lines, several T and B cell lines were screened by FACS analysis. It was observed that these monoclonal antibodies reacted with 8866, 1788, CESS and SKW-CL 4 cells, which express Fc$_\epsilon$R, but not with Daudi cells nor with the T cell lines, CEM and Molt 4, which are negative for Fc$_\epsilon$R, indicating that these monoclonal antibodies may be reacting with Fc$_\epsilon$R but not with other cell surface determinants such as Fc$_\gamma$R or Ia antigens.

Selection of hybridomas secreting antibodies with inhibitory activity on IgE-rosette formation:

Fc$_\epsilon$R on lymphocytes were detected by an essay employing fixed ox erythrocytes (ORBC) coated with human IgE (11) and the percentage of IgE-rosette-forming cells (RFC) was estimated after subtracting the percentage of non-specific RFC binding with fixed ORBC coated with BSA. For IgE-rosette inhibition assay, 25 $\mu$l of 8866 cells ($5 \times 10^6$/ml) were mixed with 100 $\mu$l hybridoma culture supernatants or control medium and incubated for 1 hr at 4°C. After incubation, 25 $\mu$l of 2 % modified ORBC coated with IgE or BSA was added, and the mixture was centrifuged at 200g for 8 min followed by incubation for 1 hr at 4°C. IgG rosette inhibition assay was performed essentially in the same manner employing Daudi cells and ORBC sensitized with IgG fraction of rabbit anti-ORBC antiserum.

Antibody purification:

Hybridoma clones, 3-5 ($\gamma$1), 8-30 ($\mu$), and 1-7 ($\gamma$2b), the first two of which were deposited at the Collection nationale de micro-organisms, Institut Pasteur (C.N.C.M), 25, rue du Dr. Roux, 75724 Paris, Cedex 15 on the 24 July 1986, under the file numbers I-583 and I-584 respectively, were selected as the possible candidates for anti-Fc$_\epsilon$R antibodies by means of their inhibitory effect on IgE-rosette formation. The necessary monoclonal antibodies were purified from ascites by 50 % saturated ammonium sulfate precipitation followed by gel filtration using Sepharose 6B (Pharmacia Fine Chemicals, Uppsala, Sweden) for IgM class or by ion exchange chromatography using QAE-Sephadex (Pharmacia Fine Chemicals) for IgG$_1$ and IgG2b classes.

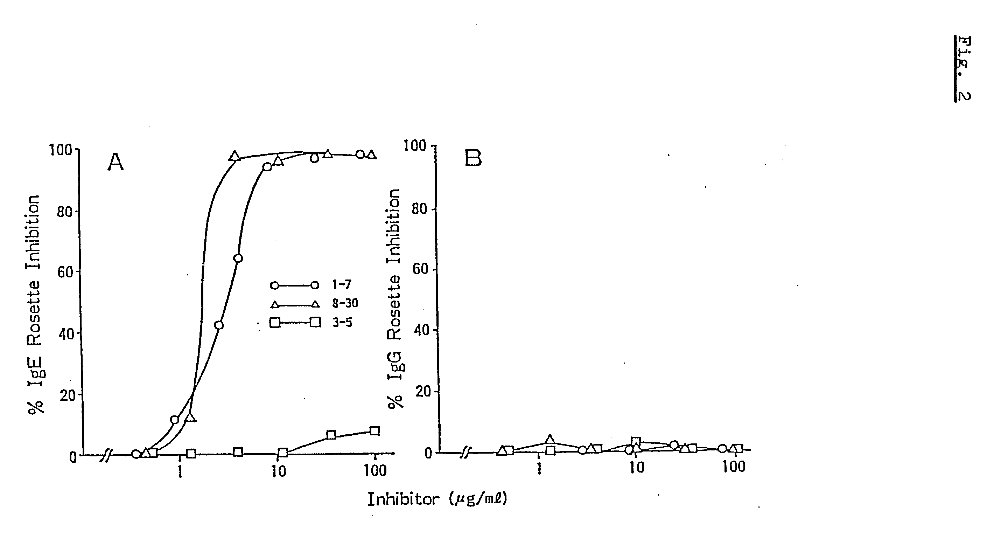

As shown in Fig 2, two monoclonal antibodies, 107 and 8-30, inhibited the rosette formation of 8866 cells with IgE-coated ox red cells (ORBC) up to 97% when used at concentrations of 5 to 10 $\mu$g/ml, while the other antibody, 3-5, showed marginal inhibitory activity only at high concentrations (100 $\mu$g/ml). The two monoclonal antibodies (1-7 and 8-30) did not inhibit the rosette formation of Daudi cells with IgG-ORBC, even when 100 $\mu$g/ml of antibodies were employed. These results prove that the monoclonal antibodies, 1-7 and 8-30, recognize Fc$_\epsilon$R, but 3-5 may recognize a different epitope of Fc$_\epsilon$R or molecules distinct from Fc$_\epsilon$R.

Preparation of human mononuclear cells (MNC):

Peripheral blood mononuclear cells were obtained from normal donors and atopic patients with bronchial asthma or atopic dermatitis but not undergoing systemic corticosteroid therapy. Serum IgE levels of these patients were between 1,000-15,000 IU.ml. Tonsillar mononuclear cells were obtained from patients with chronic tonsillitis. MNC were prepared by Ficoll-Hypaque gradient centrifugation, and B cell-enriched populations (E⁻ cells) were prepared by rosetting with (2-aminoethyl)-isothiouronium bromide (AET, Aldrich Chemical Co.) -treated sheep red blood cells.

Indirect and direct immunofluorescence analysis:

One million cells were incubated with 0,1 $\mu$g to 1 $\mu$g of biotin-conjugated monoclonal antibody for 20 min in the first step, washed several times with staining buffer (RPMI1640 minus biotin, riboflavin and phenol red/2 % FCS/10mM HEPES/0,1 % NaN₃), incubated with Texas Red (TR)-avidin for 20 min in the second step and then washed three times with staining buffer. Direct staining of cells was performed by incubating $1 \times 10^6$ cells with FITC-conjugated antibody followed by washing three times. Propidium iodide (PI, 10 $\mu$g/ml) was included in the last five minutes of the second step to label the dead cells. The one-color and two-color immunofluorescence analyses by FACS 440 and VAX computer is described in reference (12).

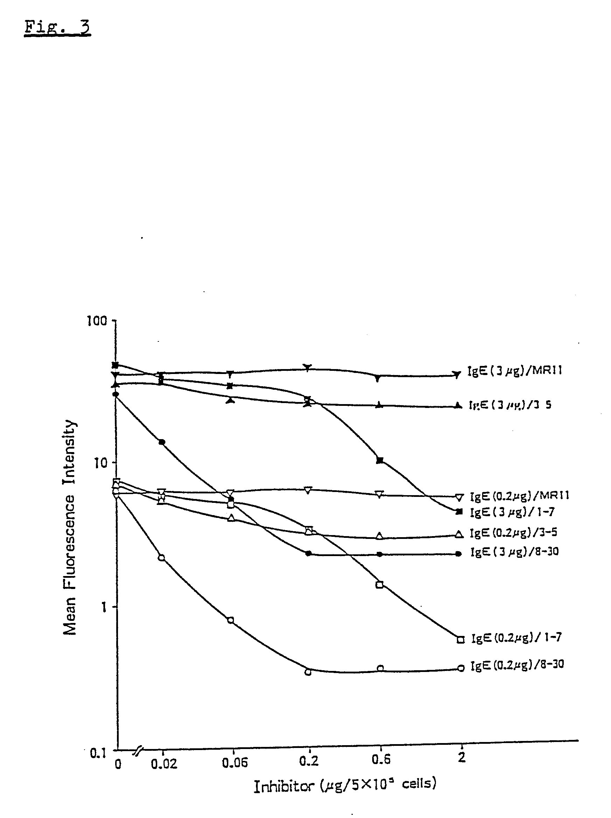

As shown in Fig. 3, both 1-7 and 8-30 monoclonal antibodies, as expected, blocked the IgE binding to 8866 cells in a dose-dependent fashion. The monoclonal antibody, 8-30, was much more effective than 1-7. This may be due to the fact that 8-30 is of IgM isotype. On the other hand, the monoclonal antibody, 3-5, could display only a slight inhibition of IgE binding at high concentrations. The control monoclonal antibody, MRII of IgM class, did not affect the binding of IgE to 8866 cells.

Inhibition assay in FACS analysis:

One half million of peripheral MNC, precultered for 1 hr and washed three times to remove cytophilic IgG or IgE, were exposed to various concentrations of monoclonal antibodies in 20 $\mu$l of staining buffer in 96 well microtiter plates at 4°C for 30 min. After incubation, cells were washed twice with staining buffer, incubated with FITC-anti BI and biotinated IgGI or IgE for 20 min, and washed twice. Subsequently, the cells were incubated with TR-avidin, washed and analyzed by FACS.

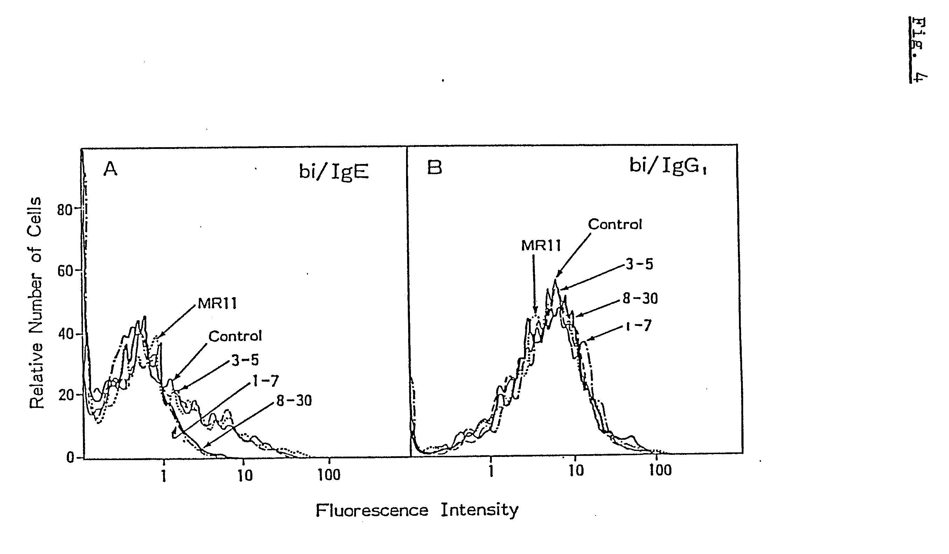

As shown in Fig 4, the binding of IgE to BI-positive cells was inhibited by the presence of either 1 $\mu$g/5 $\times 10^5$ cells of 1-7 or 8-30 antibody, but not by the monoclonal antibody, 3-5 or the control antibody, MRII. The same concentration of the monoclonal antibodies did not inhibit IgG binding to BI-positive cells. These results again demonstrated that the two monoclonal antibodies, 1-7 and 8-30, recognize Fc$_\epsilon$R and the monoclonal antibody, 3-5, might be directed to a different epitope of Fc$_\epsilon$R or to a different molecule closely associated with the receptor.

Membrane radioiodination and immunoprecipitation:

Cell surface iodinations were performed as described by Jones (16). After membrane iodination, cell lysates were prepared for immunoprecipitation according to the method of Witte and Baltimore (17). Briefly $4-10 \times 10^7$ labelled cells were lysed in 1 ml lysis buffer (18). Cellular debris were removed by centrifugation and passing through Millipore filter. The clarified supernatants were supplemented with 1 mM PMSF and 1 % aprotinin (Sigma Chemical Co., St. Louis, Mo.) and precleared overnight with human serum albumin-(HSA)-coupled Sepharose 4B beads, and subsequently incubated overnight with monoclonal antibodies or normal mouse Ig(NMIg)-coupled Sepharose 4B beads. The beads were washed three times with precipitation buffer (18) and the immunoprecipitates were eluted by boiling in electrophoresis sample buffer for 5 min. Samples were fractioned on slab gel electrophoresis according to the method of Laemmli (19) and subjected to autoradiography as described in reference (20).

Induction of Fc$_\epsilon$R expression:

Tonsillar MNC were stained with FITC-anti-BI and biotinated 8-30 antibodies and developed with TR-avidin. Fc$_\epsilon$R$^+$/BI$^+$ and Fc$_\epsilon$R$^-$/BI$^+$ cells were sorted by FACS. Sorted cells were incubated with 10 % (V/V) PHA-conditioned medium (PHA-sup) in the presence or the absence of 10 $\mu$g/ml IgE or IgGl for 3 days. After culturing for 3 days, the cells were stained with anti-BI and anti-Fc$_\epsilon$R(8-30) antibodies and reanalysed by FACS. Intensities of Fc$_\epsilon$R$^+$ B cells were plotted on the histogram by gating BI positive B cells. PHA-sup was prepared as described in reference (12). Briefly, tonsillar T cells were cultured at 1 $\times$ 10$^6$/ml with 0,1 % PHA for 48 hr and the cultured supernatant was collected. The concentrated supernatant was fractionated by HPLC gel filtration and the fraction with a M.W. of approximately 20K was employed as source of crude T cell factor(s), containing BSF-1 (BCGF), B cell differentiation factor (BCDF) and IL-2 activities.

Analysis of the determinants recognized by these monoclonal antibodies:

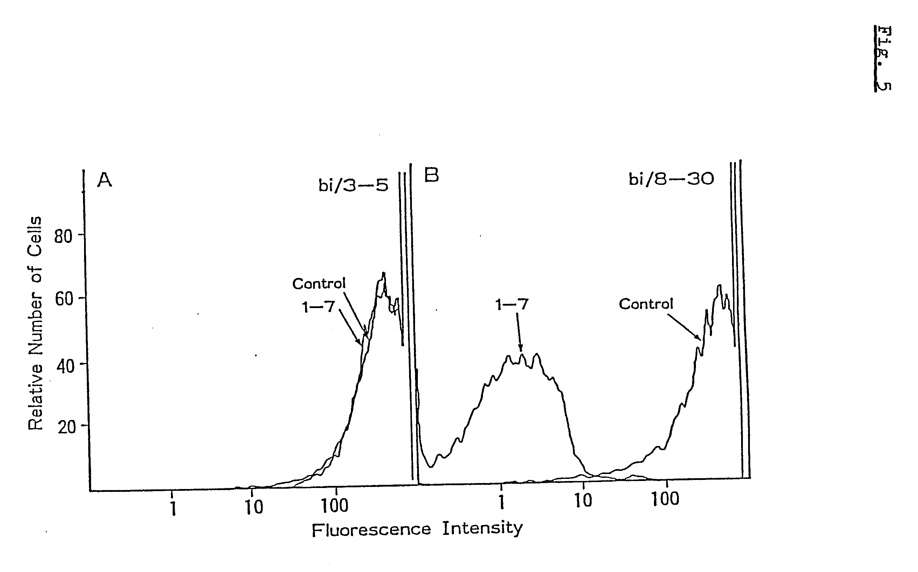

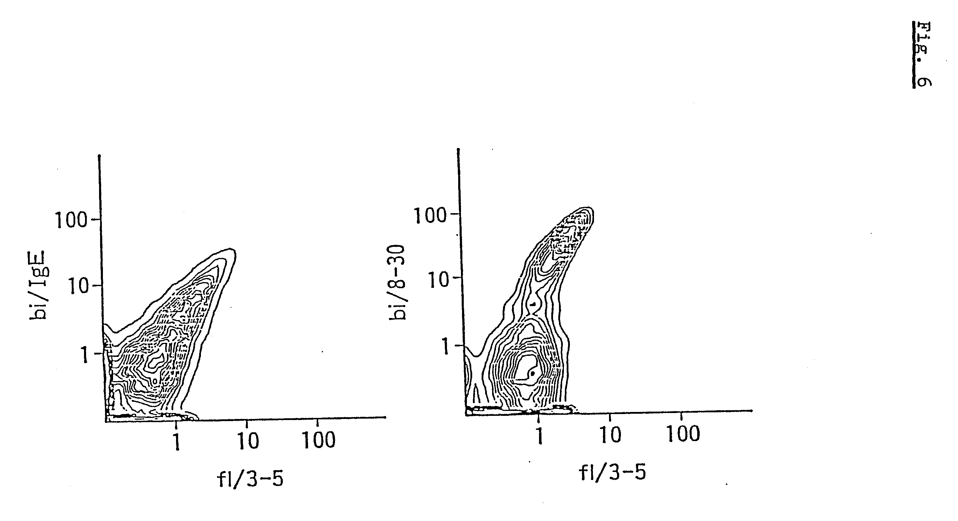

Cross staining utilizing different monoclonal antibody conjugates was employed in order to establish a correlation in the recognition of Fc$_\epsilon$R epitopes defined by these antibodies. 8866 cells were stained with biotin-3-5 or -8-30 followed by TR-avidin in the presence or abscence of the monoclonal antibody 1-7. As shown in Fig. 5, the monoclonal antibody, 1-7, could inhibit the binding of 8-30 to 8866 cells (Fig 5-B) but not the binding of 3-5 (Fig 5-A). These results demonstrate that the two monoclonal antibodies, 1-7 and 8-30, recognize the same epitope of Fc$_\epsilon$R. In order to study whether the monoclonal antibody, 3-5, recognized a different epitope of Fc$_\epsilon$R or a molecule distinct from Fc$_\epsilon$R, two color FACS analysis of tonsillar B cells with the monoclonal antibodies, 8-30 and 3-5, or IgE and 3-5, was carried out. Tonsillar B cells were stained with FITC-conjugated 3-5 antibody and biotin-conjugated 8-30 antibody or IgE followed by TR-avidin. The expression of the antigen recognized by the two monoclonal antibodies, 8-30 and 3-5 or the expression of Fc$_\epsilon$R and the antigen recognized by 3-5 was perfectly correlated (Fig 6), suggesting that the two monoclonal antibodies, 8-30 and 3-5, recognized different epitopes on the same Fc$_\epsilon$R molecules.

Molecules recognized by these monoclonal antibodies:

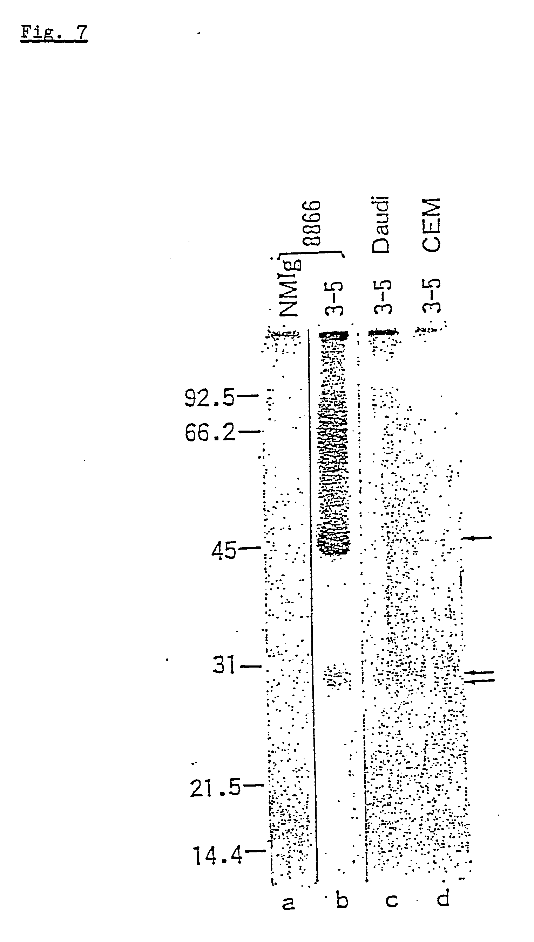

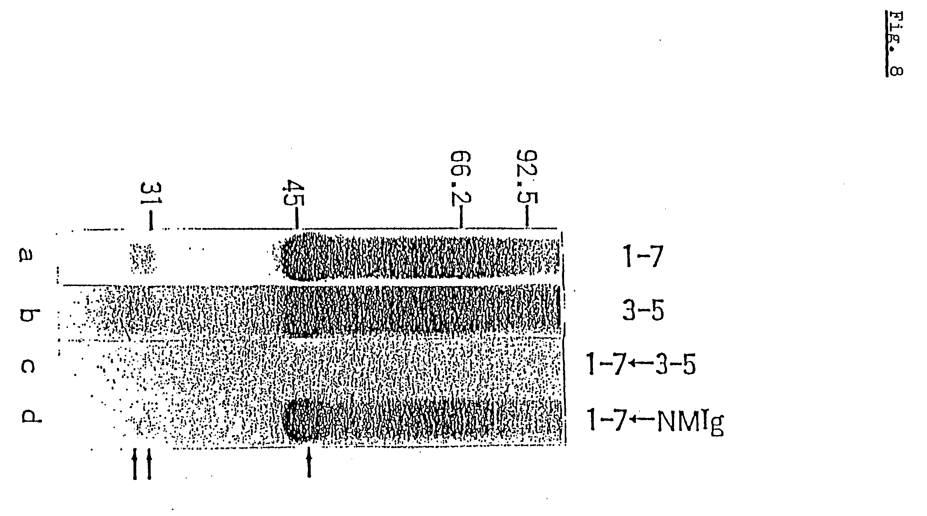

In order to analyze the molecules recognized by these monoclonal antibodies, Fc$_\epsilon$R-positive 8866 and Fc$_\epsilon$R-negative Daudi and CEM cells were surface labelled with $^{125}$I and lysed in lysis buffer containing 0,5 % NP-40. After preabsorption with HSA-Sepharose, lysates were immunoprecipitated with the monoclonal antibody, 1-7 or 3-5, or normal mouse Ig (NMIg), and analyzed by SDS-PAGE (Fig 7 and Fig 8). In the lysates of 8866 cells, one major band with the M.W of 46 Kd and one or two minor bands with the M.W of 25-29 Kd as well as broad smear between 60 to 90 Kd were detected under reducing and non-reducing conditions, whereas lysates from Daudi cells or CEM cells did not show any bands with the monoclonal antibodies. In order to study whether molecules recognized by two monoclonal antibodies, 1-7 and 3-5, were identical or not, sequential immunoprecipitation was carried out. Cell lysates of $^{125}$I-labelled 8866 cells were immunoprecipitated with either 3-5 moncolonal antibody or NMIg, and effluents were further immunoprecipitated with the monoclonal antibody, 1-7. The analysis by SDS-PAGE is shown in Fig 8. Both monoclonal antibodies, 1-7 and 3-5, precipitated 46 Kd molecules (Fig. 8 lane a and b). Preclearing with the monoclonal antibody, 3-5, virtually eliminated the molecules recognized by the monoclonal antibody, 1-7, (Fig. 8 lane c) but preclearing with NMIg did not affect the reactivity of the monoclonal antibody, 1-7, (Fig 8 lane d) indicating that molecules recognized by the two monoclonal antibodies, 1-7 and 3-5, were identical. The results demonstrate that all the three monoclonal antibodies, 1-7, 3-5 and 8-30, recognize Fc$_\epsilon$R on human B lymphocytes.

Expression of Fc$_\epsilon$R on peripheral and tonsillar B lymphocytes:

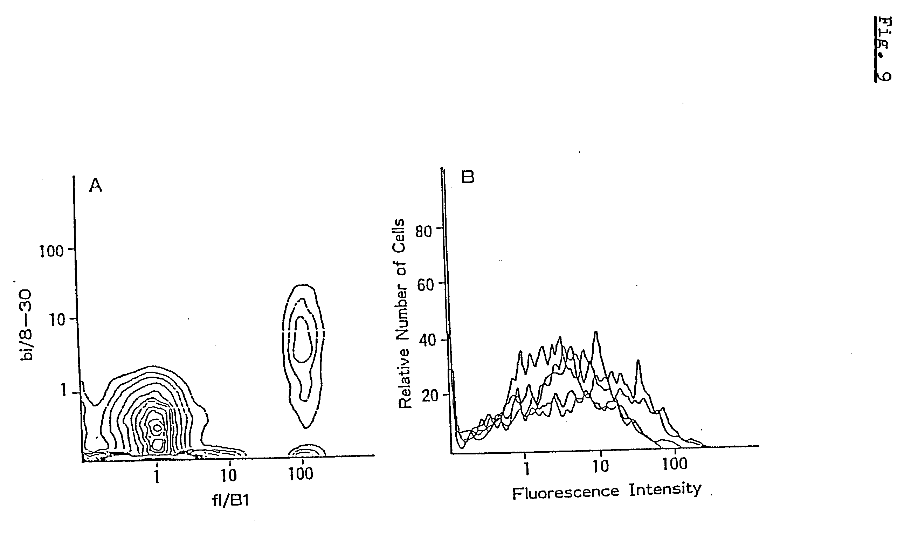

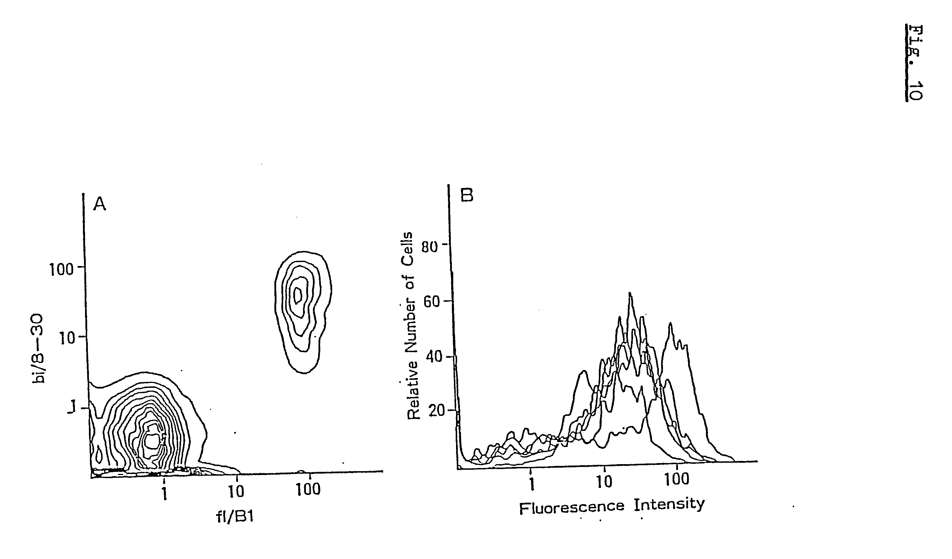

By utilizing the monoclonal antibodies, 3-5 and 8-30, the expression of Fc$_\epsilon$R on the PBL from normal individuals and atopic patients was analyzed. Representative results of FACS analysis are depicted in Fig 9. Approximately 50 % (40-60 %) of BI-positive B cells were stained with the 8-30 monoclonal antibody (Fig 9-A). Essentially the same pattern was observed with the 3-5 monoclonal antibody. Frequency of Fc$_\epsilon$R$^+$ population of several normal individuals was depicted in Fig 9-B. In the PBL from atopic patients with elevated serum IgE level (1,000-15,000 IU/ml), almost all the BI-positive B cells were positive for the

5

expression of $Fc_\epsilon R$, and the fluorescence intensity was much higher than that of normal B cells (Fig 10-A.B), showing increases not only in the number of $Fc_\epsilon R$-positive B cells but also in the density of $Fc_\epsilon R$ on the B cells in atopic patients. However, as shown in two color FACS analysis of Fig 9 and Fig 10, the expression of $Fc_\epsilon R$ on T cells was not detectable in atopic or normal individuals even when a sensitive FACS analysis was employed.

## Induction and augmentation of $Fc_\epsilon R$ expression:

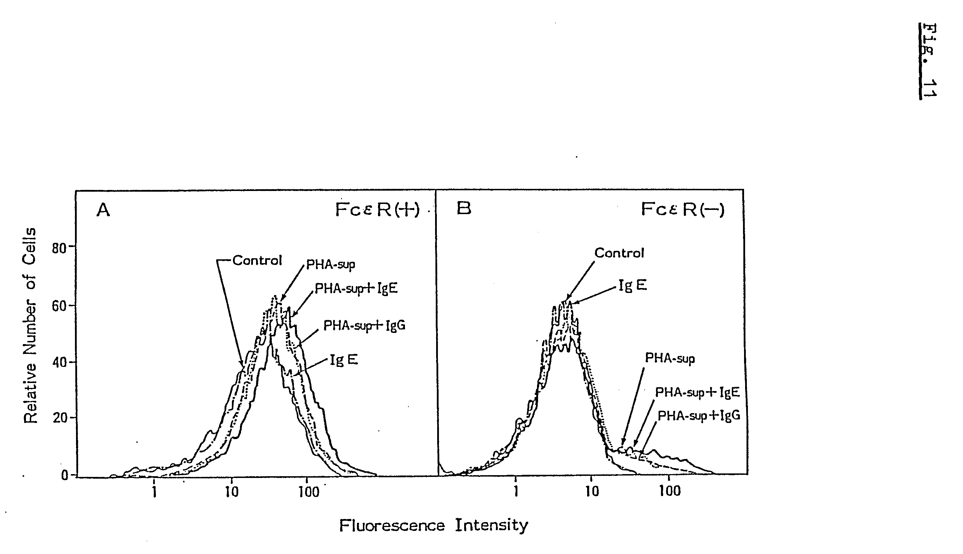

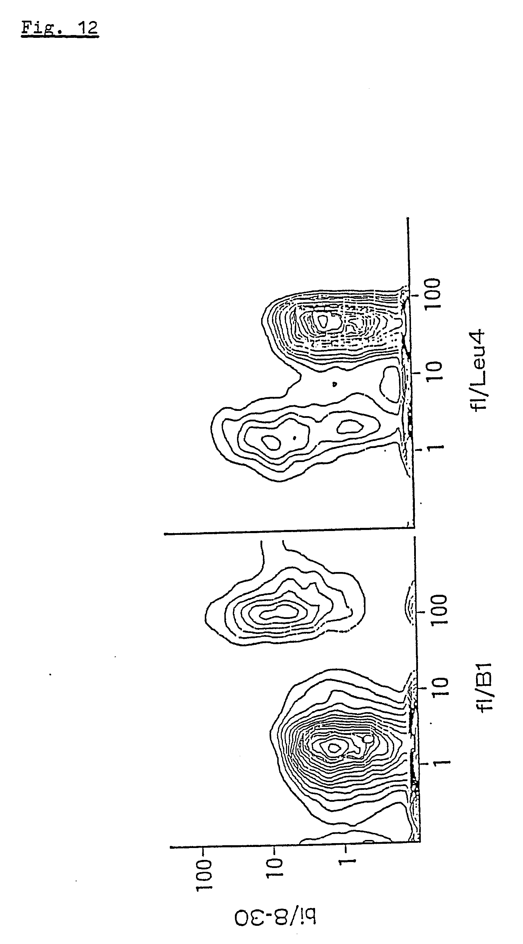

The previous studies showed that the stimulation of lymphocytes with PHA-conditioned medium and IgE could induce $Fc_\epsilon R$ expression detectable by rosette formation (21). Therefore, attempts were made to assess $Fc_\epsilon R$ induction by the monclonal anti-$Fc_\epsilon R$ antibodies. As described above, $Fc_\epsilon R$ was expressed exclusively on B cells, but not on all B cells. $Fc_\epsilon R$-positive and $Fc_\epsilon R$-negative B cells were sorted from tonsillar MNC by a monoclonal antibody, 3-5 and FACS and each population was incubated with or without PHA-conditioned medium (PHA-sup) in the presence or absence of IgE. As shown in Fig 11, incubation with PHA-sup induced an increase in the expression of $Fc_\epsilon R$ on the $Fc_\epsilon R$-positive B cell population when detected by a monoclonal antibody, 8-30. An increase in the density of $Fc_\epsilon R$ was markedly augmented in the presence of IgE but not of IgG. On the other hand, the same stimulation did not induce the expression of $Fc_\epsilon R$ on the $Fc_\epsilon R$-negative B cell population. Essentially the same result was observed with the monoclonal antibody, 3-5. The presence of IgE enhanced the expression of $Fc_\epsilon R$ on the $Fc_\epsilon R$-positive B cells. However IgE alone neither induced nor augmented $Fc_\epsilon R$ expression on the $Fc_\epsilon R$-positive or $Fc_\epsilon R$-negative B cells. Inducibility of $Fc_\epsilon R$ on T cells was also examined. PBL from normal donors were stimulated with or without PHA-sup plus IgE and analyzed by FACS. An increase in $Fc_\epsilon R$ expression was observed solely in B cells, but no $Fc_\epsilon R$ induction was detected in T cell population even after 8 days of the culture, when assessed either with 8-30 or 3-5. Mixed lymphocyte reactions in the presence of IgE also failed to induce $Fc_\epsilon R$ (Fig 12). The analysis on cultured tonsillar cells showed essentially the same result. Taken collectively, these results indicate that $Fc_\epsilon R$ can be induced on some B cell subpopulations, while T cells do not express $Fc_\epsilon R$ on their surface even after the induction.

A further object of the present invention is the use of the before mentioned antibodies to differentiate bone marrow B cells and circulating B cells showing that $Fc_\epsilon R$ is a B cell-specific surface molecule:

## Expression of $Fc_\epsilon R$ on human B lymphocytes from peripheral blood (PBL), tonsils and bone marrow:

By employing the new monoclonal anti-$Fc_\epsilon R$ antibodies, 1-7 ($\gamma_{2b}$), 3-5 ($\gamma_1$) and 8-30 ($\mu$) the distribution of $Fc_\epsilon R^+$ cells in various lymphoid tissues, such as peripheral blood (PBL), tonsils and bone marrow was examined. As the three monoclonals showed the same results, the monoclonal antibody, 8-30, was employed in all the experiments. MNC from various lymphoid tissues were stained with FITC-anti-Bl and biotinated anti-$Fc_\epsilon R$ followed by TR-avidin and analysed by dual laser FACS. As described in Table 1, $Fc_\epsilon R$ was expressed on most $Bl^+$-B cells in PBL and approximately one half of $Bl^+$-B cells derived from tonsils. On the other hand, only a small fraction of $Bl^+$-B cells were positive for the $Fc_\epsilon R$ expression in bone marrow. It is noteworthy to point out that $Bl^-$-cells did not express $Fc_\epsilon R$ in any of the lymphoid tissues tested. The variability in the frequency of $Fc_\epsilon R^+$ cells in PBL, tonsils and bone marrow proves that $Fc_\epsilon R$ may be expressed on B cells at a certain defined differentiation or activation stage.

## Correlation of the $Fc_\epsilon R$ expression and Ig-isotypes on B cells:

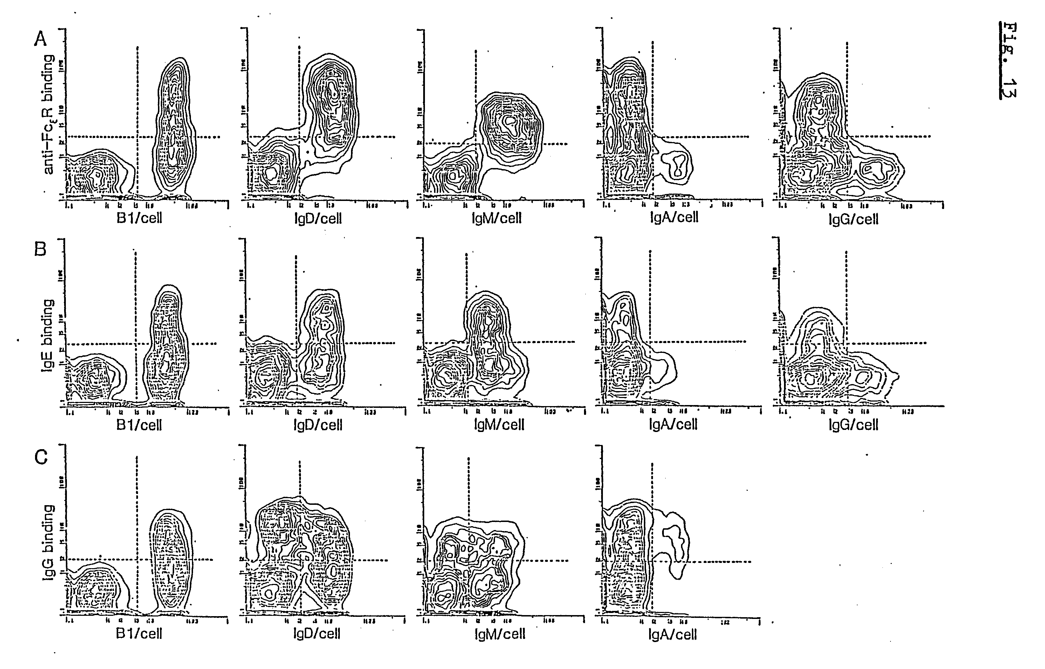

Tonsillar B cells were stained with anti-$Fc_\epsilon R$ and with conjugates directed against different Ig-isotypes. As shown in Fig. 13 (A), $Fc_\epsilon R$ was expressed on most $\mu$ and $\delta$ positive B cells in tonsils. On the other hand, almost all $\gamma$-or $\alpha$-bearing B cells, which were abundantly observed in tonsils, did not express $Fc_\epsilon R$ on their surface. Other experiments were conducted in order to study whether the $Fc_\gamma R$ expression was also correlated with the isotype expression as observed with the $Fc_\epsilon R$. Since antibodies specific to human $Fc_\gamma R$ were not available, biotinated IgE and IgG were employed for the comparison of the expression of $Fc_\gamma R$ and $Fc_\epsilon R$ on $\mu^+\delta^+$ B cells and on $\gamma^+$ or $\alpha^+$ B cells. As shown in Fig. 13 (A) and (B), the specificity of the binding of biotinated IgE was almost comparable to that of the anti-$Fc_\epsilon R$ antibody, in fact, the IgE-binding was observed on $\mu^+\delta^+$ B cells but not on $\gamma^+$ or $\alpha^+$ B cells. On the other hand, almost all $\alpha^+$ B cells could

bind IgG$_1$ and some of $\mu^+\delta^+$ B cells were negative for the expression of Fc$\gamma$R, indicating that the Fc$\gamma$R expression was not correlated with immunoglobulin isotype expression. Approximately 50 % $\mu^+$ or $\delta^+$ cells could not bind IgE although they were stained with anti-Fc$_\epsilon$R antibody which is due to the difference of the affinity of IgE and anti-Fc$_\epsilon$R antibodies to Fc$_\epsilon$R.

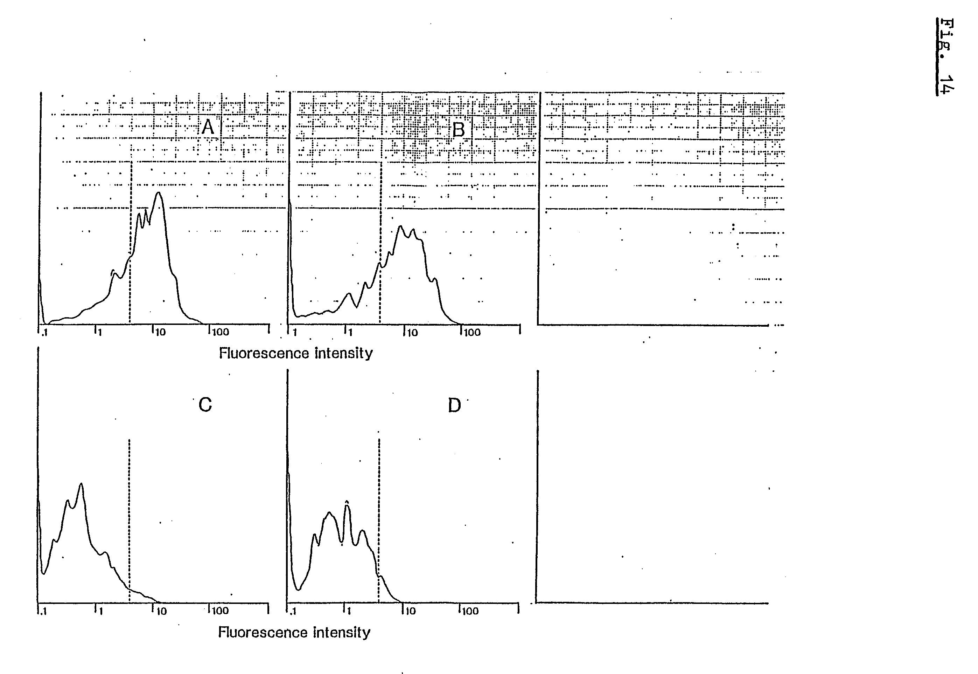

Most peripheral blood B cells were $\mu^+\delta^+$ and this may explain why most BI$^+$-cells in PBL are positive for the Fc$_\epsilon$R expression, while only 50 % of tonsillar B cells were positive for this marker. It is therefore difficult to analyze the expression of Fc$_\epsilon$R on $\mu^+$ or $\alpha^+$ B cells in PBL on the contour plot because of the paucity of these cells. Fig. 14 shows the histograms of the Fc$_\epsilon$R expression on $\mu^+$, $\delta^+$, $\gamma^+$ or $\alpha^+$ B cells in PBL. Most $\delta^+$ (A) or $\mu^+$ (B) B cells expressed Fc$_\epsilon$R as in the case of tonsillar B cells. However, $\gamma^+$ (C) or $\alpha^+$ (D) B cells did not express Fc$_\epsilon$R, although a small fraction of these cells were found to show a weak expression. The results show that Fc$_\epsilon$R is preferentially expressed on circulating $\delta^+$ and $\mu^+$ B cells but not on $\gamma^+$ or $\alpha^+$ B cells and that may explain the reason why PBL includes more Fc$_\epsilon$R$^+$ cells than tonsils.

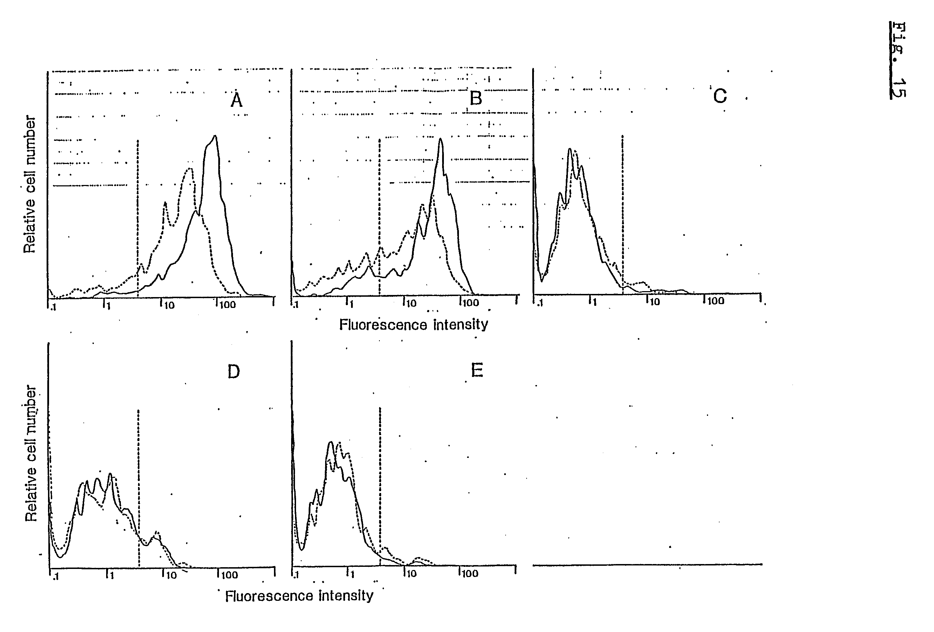

Because of the paucity of $\epsilon$-bearing B cells in PBL or tonsils, the expression of Fc$_\epsilon$R on $\epsilon^+$ B cells could not be examined. Therefore, PBL from patients with hyper IgE-syndrome were employed. Fig. 15 demonstrates the representative results observed in PBL from two patients with hyper IgE-syndrome. In fact, the Fc$_\epsilon$R expression was augmented in $\mu^+$ or $\delta^+$ B cells in these patients compared to that in normal individuals (Fig. 14, A and B). However, $\gamma^+$ or $\alpha^+$ B cells did not express Fc$_\epsilon$R even in PBL from patients with hyper IgE-syndrome (Figs. 2, C and D). It is worth noting that Fc$_\epsilon$R expression was not observed in $\epsilon^+$ B cells (Fig. 14, E). All of these results indicate that the Fc$_\epsilon$R expresssion is observed only on mature $\mu^+$ $\delta^+$ cells and the expression is lost after the isotype switching from $\mu^+$ $\delta^+$ B cells to $\gamma^+$, $\alpha^+$ or $\epsilon^+$ B cells occurs.

<u>Induction of Fc$_\epsilon$R in bone marrow and tonsillar B cells:</u>

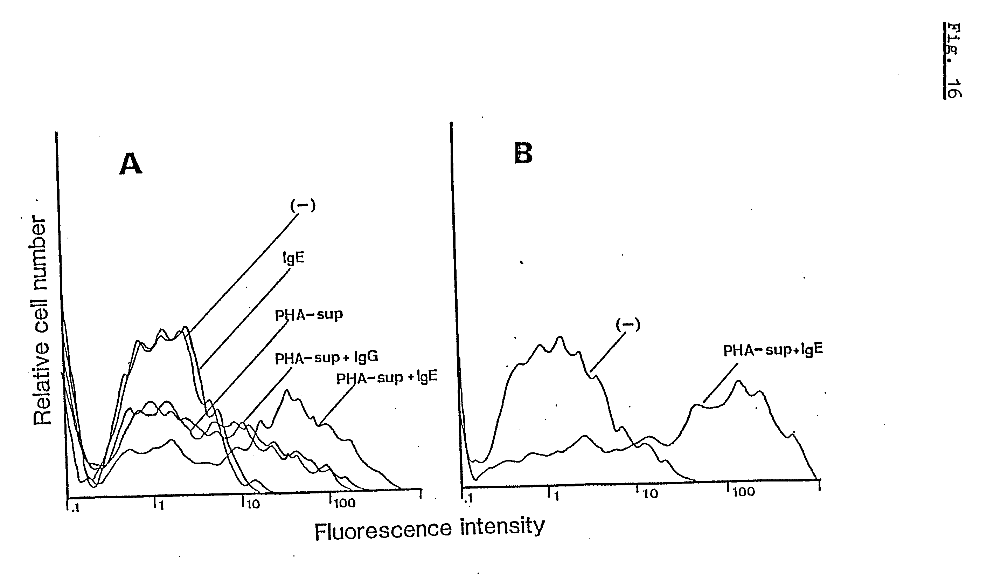

As described in Table 1, $\mu^+$ $\delta^+$ B cells in PBL were Fc$_\epsilon$R-positive, while B cells in bone marrow were negative, suggesting that only circulating mature B cells are able to express Fc$_\epsilon$R. Moreover experiments were conducted to study whether Fc$_\epsilon$R could be induced in bone marrow B cells by certain factors. As shown in Fig. 16, T cell-derived PHA-conditioned medium (PHS-sup) could induce the Fc$_\epsilon$R expression on BI$^+$-B cells (Fig 16A), particularly on $\delta^+$ B cells (Fig 16B). The addition of IgE but not IgG could augment the PHA-sup-induced expression of Fc$_\epsilon$R. However, IgE alone without PHA-sup did not show any effect on the Fc$_\epsilon$R expression.

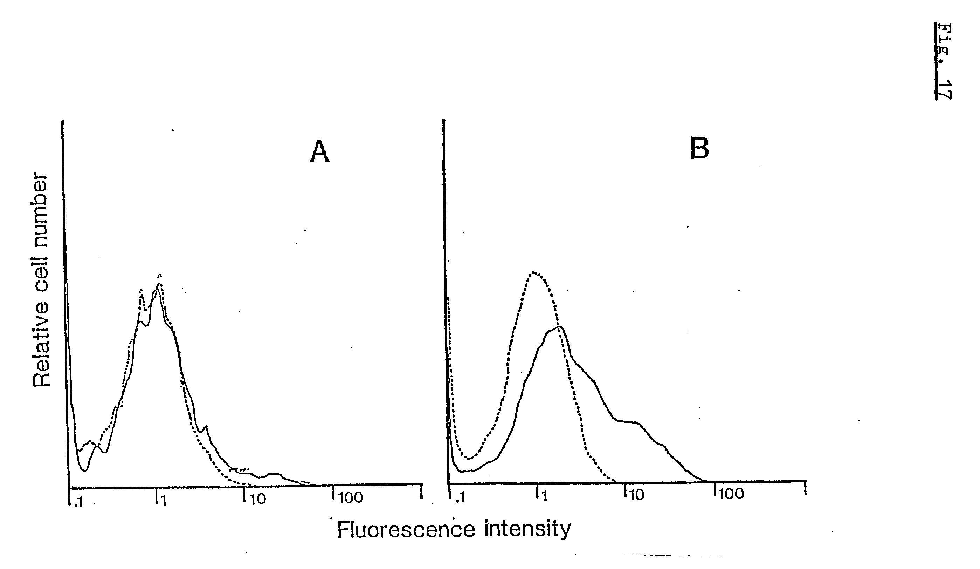

Circulating $\delta^-$ B cells, which are mainly $\alpha^+$ or $\gamma^+$, do not express Fc$_\epsilon$R. In order to study whether Fc$_\epsilon$R is inducible in those cells, $\delta^-$ B cells were sorted from tonsillar BI$^+$ cells and incubated with PHA-sup and IgE. As shwon in Fig. 17(A), the stimulation did not induce any expression of Fc$_\epsilon$R on circulating $\delta^-$ B cells, indicating that the loss of the Fc$_\epsilon$R expression was irreversible in the cells which had already completed isotype switching. A small fraction of $\delta^+$ B cells in tonsil did not express Fc$_\epsilon$R. However, as shown in Fig. 17(B), PHA-sup plus IgE could induce the Fc$_\epsilon$R expression in these cells, suggesting that they may be in an immature stage of differentiation. Therefore, these results indicate that there are two different varieties of Fc$_\epsilon$R-negative B cells: immature B cells and B cells which had already switched to other isotypes.

<u>Failure of the Fc$_\epsilon$R expression on lymphocytes from patients with certain immunodeficiencies:</u>

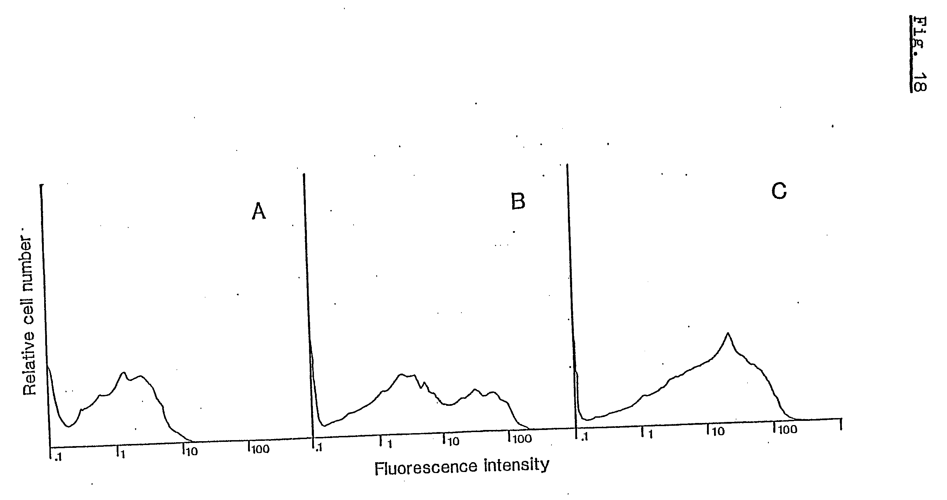

PBL from patients with ataxia teleangiectasia (AT) or common variable immunodeficiencies (CVI) were stained with anti-Fc$_\epsilon$R and anti-BI or anti-IgM and anti-IgD and analysed by FACS. Two cases of CVI and two cases of AT were studied and the results are summarized in Table 2. In all these cases, most of the B cells expressed both IgM and IgD, although a patient (AT-2) had few B cells. It was interesting to note that more than 90 % of BI$^+$-B cells in all these patients did not express Fc$_\epsilon$R. The results suggest that B cells from these patients are similar in their differentiation stage to $\mu^+$ $\delta^+$ B cells found in the bone marrow rather than in PBL. The results furthermore suggest a possible arrest in the maturation of these B cells. In order to study whether Fc$_\epsilon$R could be induced by external signals, PBL from a patient with CVI (CVI-1) were cultured with PHA-sup and IgE for 3 days and the Fc$_\epsilon$R expression on B$^+$-B cells was examined. As shown in Fig. 18, PHA-sup could induce the Fc$_\epsilon$R expression in a certain fraction of those B cells and the addition of IgE would augment the expression, suggesting that those B cells are in a similar differentiation stage as that of bone marrow B cells.

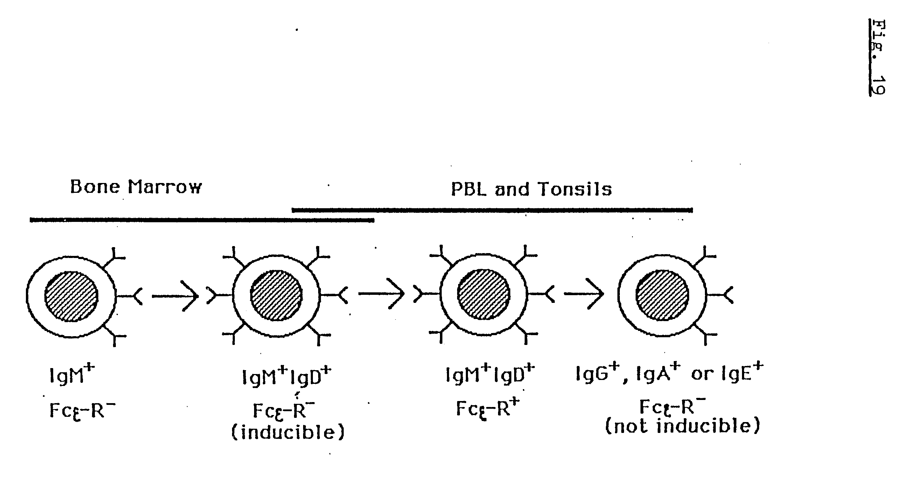

The before mentioned results show that $Fc_\epsilon R$ is a B cell-specific surface molecule, the expression of which is strictly correlated with the differentiation stage of B cells; i) bone marrow B cells with surface IgM and IgD do not express $Fc_\epsilon R$, although its expression could be induced, ii) circulating B cells with surface IgM and IgD express $Fc_\epsilon R$, but iii) circulating B cells with no surface IgD, which have already undergone isotype-switching into IgG, IgA or IgE bearing cells do not express and can not be induced to express $Fc_\epsilon R$. Therefore, according to these observations, the differentiation of B cells can be divided into three stages as described in Fig. 19:

i) $\mu^+\delta^+ Fc_\epsilon R^-$ bone marrow B cells,

ii) $\mu^+\delta^+ Fc_\epsilon R^+$ circulating B cells and

iii) $\mu^+\delta^+ Fc_\epsilon R^-$ with $\gamma^+$, $\alpha^+$ or $\epsilon^+$ B cells.

By making use of the fact that each of the monoclonal antibodies, for example the antibodies 3-5 and 8-30, recognize a different epitope on the same receptor, it was possible to establish an ELISA for detection of $Fc_\epsilon R$-receptors. The Elisa according to the invention comprises usually a solid phase carrying a monoclonal antibody to said surface receptor for IgE, e.g. a carrier coated with monoclonal antibody 3-5 and/or 8-30, and a second a monoclonal antibody to said surface receptor for IgE bound to a detectable label such as a radioactive isotope, an enzyme, a fluorescent or chemilu miniscent compound or a second antibody specifically binding to an above mentioned antibody and carrying any one of the afore-mentioned labels, e.g. alkaline phosphatase-conjugated anti-mouse IgM, preferably alkaline phosphatase-conjugated 8-248 anti-mouse IgM:

The monoclonal antibody 3-5 is of $\gamma_1$ class, does not significantly inhibit the binding of IgE, and binds to a specific epitope of $Fc_\epsilon R$.

The monoclonal antibody 8-30 is of $\mu$ class and inhibits the binding of IgE and binds to a specific epitope of $Fc_\epsilon R$.

Therefore, in the new ELISA the above mentioned monoclonal antibodies bind in combination selectively to $Fc_\epsilon R$ found in culture supernatants or lysates of RPMI8866 cells, EB-transformed cells and serum derived from atopic and non-atopic individuals. The double antibody sandwich technique utilizing the monoclonal antibodies 3-5 and 8-30 identifies the presence of $Fc_\epsilon R$-receptors in serum from atopic and non-atopic individuals and enables the state of hypersensitivity of an individual to be followed. This technique is a potential diagnostic tool in allergy since, as in the case of determining the level of IgE in serum, it allows monitoring and diagnosis of an allergic condition.

Technical detail

Ninety-six well microtiter plates are coated with monoclonal antibody 3-5 in coating buffer, 100μl of 10 μg/ml. The plates are incubated overnight at 4°C, and washed with rinse buffer 4 times before use. 100 μl of test sample is added to each well and incubated for 2 hours at room temperature (the test sample can be diluted first with diluent buffer to allow a titration). The test samples are dumped and the plated washed 4 times with rinse buffer followed by the addi tion of 100 μl of second monoclonal antibody, 8-30 diluted to 1 μg/ml in diluent buffer and incubated for 2 hours at room temperature. The wells are washed 4 times with rinse buffer followed by the addition of 100 μl of goat anti-mouse IgM conjugated to alkaline phosphatase (pretitrated) and incubated for 2 hours at room temperature. The wells are washed with rinse buffer and 200 μl of 1 mg/ml of substrate, p-Nitrophenyl Phosphate Disodium added. The enzymatic reaction is allowed to proceed and the optical (color) determination measured at 405 and 620 nm.

Table 1

Expression of $Fc_\varepsilon R$ on B cells from peripheral blood, tonsils and bone marrow [a]

| | $Fc_\varepsilon R$ positive cells | | |
|---|---|---|---|
| | total % [b] | % in $B1^+$ cells [c] | % in $B1^-$ cells [c] |
| PBL | 11.2 ± 3.1 | 87 ± 5.2 | < 0.1 |
| tonsils | 28.4 ± 7.4 | 51 ± 11.3 | < 0.1 |
| bone marrow | 0.5 ± 0.2 | 3.0 ± 1.2 | < 0.1 |

a) PBL from 5 donors, tonsillar MNC from 5 donors and bone marrow MNC from 3 donors were stained with FITC-anti-B1 and biotinated anti-$Fc_\varepsilon R$, developed with TR-avidin and analyzed by FACS.

b) Mean percentage of positive cells ± S.D.

c) Mean percentage of $Fc_\varepsilon R^+$ cells in $B1^+$ or $B1^-$ cells assayed by two color analysis as described in Materials and Methods.

Table 2

Failure of Fc$_\xi$R expression on lymphocytes from
immunodeficiency patients

| | CVI-1 | CVI-2 | CVI-3 | AT-1 | AT-2 |
|---|---|---|---|---|---|
| B1[a] | 20 | 2.5 | 4 | 30 | 5 |
| IgD[a] | 17 | 1.8 | 2 | 27 | 2 |
| IgM[a] | 19 | 2.3 | 3.6 | 28 | 4.5 |
| Fc$_\xi$R[a] | 0.5 | 0.1 | 0.2 | 0.8 | 0.05 |
| Fc$_\xi$R/B1$^{+}$[b] | 2.5 | 4 | 5 | 2.4 | 1.0 |

a) PBL from patients with CVI or AT were stained with anti-B1,
anti-IgD, anti-IgM or anti-Fc$_\xi$R and analyzed by FACS. Per-
centages of positive cells were shown.

b) Cells were stained FITC-anti-B1 and biotinated Fc R and
analyzed by FACS. The percentages of Fc$_\xi$R$^{+}$ cells in
B1$^{+}$ cells were assayed by two colour analysis with FACS as
described in Materials and Methods.

References

1. Kishimoto, T., and K. Ishizaka. 1973. Regulation of antibody response in vitro. VI. Carrier-specific helper cells for IgG and IgE antibody response. J. Immunol., 111:720.

2. Kishimoto, T., Y. Hirai, M. Suemura, and Y. Yamarua. 1976. Regulation of antibody response in different immunoglobulin classes. I. Selective suppression of anti-DNP IgE antibody response by preadministration of DNP-coupled mycobacterium. J. Immunol., 117:396.

3. Kishimoto, T. 1982. IgE class-specific suppressor T cells and regulation of the IgE response. Prog. Allergy., 32:265.

4. Ishizaka, K. 1984. Regulation of IgE synthesis. Ann. Rev. Immunol., 2:159.

5. Katz, D.H. 1985. The IgE antibody system is coordinately regulated by FcR epsilon-positive lymphoid cells and IgE-selective soluble factors. Int. Archs Allergy appl. Immunol., 77:21.

6. Deguchi, H., M. Suemura, A. Ishizaka, Y. Ozaki, s. Kishimoto, Y. Yamamura, and T. Kishimoto. 1983. IgE class-specific suppressor T cells and factors in humans. J. Immunol., 131:2751.

7. Suemura, M., A. Ishizaka, S. Kobatake, K. Sugimura, K. Maeda, K. Nakanishi, S. Kishimoto, Y. Yamamura, and T. Kishimotor. 1983. Inhibition of IgE production in B hybridomas by IgE class-specific suppressor factor from T hybridomas. J. Immunol., 130:1056.

0 254 767

8. Suemura, M., J. Yodoi, M. Hirashima, and K. Ishizaka. 1980. Regulatory role of IgE-binding factors from rat T lymphocytes. I. Mechanism of enhancement of IgE response by IgE-potentiating factor. J. Immunol., 125:148.

9. Hirashima, M., J. Yodoi, and K. Ishizaka. 1980. Regulatory role of IgE-binding factor from rat T lymphocytes. III. IgE specific suppressive factor with IgE-binding activity. J. Immunol., 125:1442.

10. Uede, T., T.F. Huff, and K. Ishizaka. 1984. Suppression of IgE suhthesis in mouse plasma cells and B cells by rat IgE-suppressive factor. J. Immunol., 133-803.

11. Gonzalez-Molina, A., and H.L. Spiegelberg. 1977. A sub-population of normal human peripheral B lymphocytes that bind IgE. J. Clin. Invest., 59:616.

12. Kikutani, H., T. Kimura, J. Nakamura, R. Sato, A. Muraguchi, N. Kawamura, R.R. Hardy, and T. Kishimoto. 1986. Expression and function of an early activation marker restricted to human B cells. J. Immunol., in press

13. Goding, J. 1976. Conjugation of antibodies with fluorochromes. Modification to standard method. J. Immunol. Methods., 13:215.

14. Bayer, E.A., and M. Wilcheck. 1978. The avidin-biotin complex as a tool in molecular biology. Trends Biochem. Sci., 3:N257.

15. Maruyama, S., T. Naito, K. Kakita, S. Kishimoto, Y. Yamamura, and T. Kishimoto. 1983. Preparation of a monoclonal antibody against human monocyte lineage. J. Clin. Immunol., 3:57.

16. Jones, P.P. 1980. Analysis of radiolabeled lymphocytes proteins by one-and two-dimensional polyacrylamide gel electrophoresis. In Selected Methods in Cellular Immunology, edt. by B.B. Mishell, and S.M. Shiigi, W.H. Freeman and Company, San Francisco, pp. 398-440.

17. Witte, O.N., and D. Baltimore, 1978. Relationship of retrovirus polyprotein cleavages to virion maturation studied with temperature-sensitive murine leukemia virus mutants. J. Virol., 26:750.

18. Rothenberger, E., and E.A. Boyse. 1979. Synthesis and processing of molecules bearing thymus leukemia antigen. J. Exp. Med., 150:777.

19. Laemmli, U.K. 1970. Cleavage of structural proteins during the assembly of the head of bacteriophage T4. Nature, 227:680.

20. Yoshida, N., T. Watanabe, N. Sakaguchi, H. Kikutani, S. Kishimoto, Y. Yamamura, and T. Kishimoto. 1982. Induction of 19S IgM secretion in a murine pre-B cell line, 7OZ/3, by cell hybridization with nonsecreting myeloma cells. Mol. Immunol., 19:1415.

21. Ishizaka, K., and K. Sandberg. 1981. Formation of IgE-binding factors by human T lymphocytes. J. Immunol., 126:1692.

## Legends for Figures

### Fig. 1

FACS analysis of 8866, Daudi and CEM cells with monoclonal, anti-Fc$_\epsilon$R antibodies (1-7, 3-5 and 8-30). Cells were incubated with culture supernatants of the hybrid clones followed by staining with FITC-goat-anti-mouse IgM + IgG antibodies and applied to FACS analysis.

### Fig. 2

Inhibitory activity of the monoclonal anti-Fc$_\epsilon$R antibodies on IgE (panel A) and IgG (panel B) rosette formation. For IgE-rosette assay, 8866 cells were preincubated with varying concentrations of the monoclonal antibodies or control medium and subsequently incubated with fixed ORBC coated with IgE or BSA. IgG-rosette assay was carried out employing Daudi cells and rabbit IgG-coated ORBC.

### Fig. 3

Blocking of IgE binding to 8866 cells by the monoclonal antibodies. 8866 cells were stained with either 3 $\mu$g or 0.2 $\mu$g/5$\times$10$^5$ cells of biotin-IgE followed by TR-avidin in the presence or absence of varying concentrations of the monoclonal antibodies (1-7, 3-5 and 8-30) or a B cells-specific monoclonal antibody (MRII) and applied to FACS analysis. The mean fluorescence intensity was calculated by VAX-11 computer.

Fig. 4

Blocking of IgE binding but not IgG binding to peripheral MNC by the monoclonal antibodies. Peripheral MNC were pre-incubated with either 1 $\mu$/5×10$^5$ cells of the anti-Fc$_\epsilon$R monoclonal antibodies or a B cell specific antibody (MRII). After washing, cells were incubated with FITC-anti-BI and 3 $\mu$g/5×10$^5$ cells of biotinated IgE or IgG$_1$ followed by TR-avidin and analyzed by FACS. Relative number of IgE (panel A) or IgG (panel B) binding cells to BI positive cells (B cells) was shown in the histograms.

Fig. 5

Inhibition of the binding of the monoclonal antibody 8-30 but not 3-5 to 8866 cells by the monoclonal antibody, 1-7. 8866 cells were stained with 1 $\mu$g/1×10$^6$ cells of biotin-3-5(A) or 0.1 $\mu$g/1×10$^6$ cells of biotin-8-30(B) followed by TR-avidin in the presence or absence of 9 $\mu$g/1×10$^6$ cells of 1-7 and analysed by FACS.

Fig. 6

Correlated expression of Fc$_\epsilon$R and the antigen recognized by the monoclonal antibody, 3-5 or 8-30. Tonsillar B cells were stained with 0.5 $\mu$g/1×10$^6$ cells of FITC-labelled 3-5 and 0.1 $\mu$g/1×10$^6$ cells of biotin-labelled 8-30 or 3 $\mu$g/1×10$^6$ cells of biotinated IgE followed by TR-avidin and the two color FACS analysis was carried out.

Fig. 7

SDS-PAGE analysis of the surface molecules reactive with the monoclonal antibody (3-5). 8866 (lane a and b), Daudi (lane c) and CEM (lane d) were surface-labelled with $^{125}$I and cell lysates were immunoprecipitated with either the mono clonal antibody (3-5) (lane b through d) or NMIg (lane a). The precipitates were analysed by SDS-PAGE under non-reducing condition. (Identical results were obtained under reducing condition).

Fig. 8

Sequential immunoprecipitation of the surface molecules of 8866 cells with the monoclonal antibodies 3-5 and 1-7. 8866 cells were surface-labelled with $^{125}$I and cell lysates were immunoprecipitated with 1-7 (lane a) or 3-5 (lane b). After precipitation with 3-5 or NMIg, the supernatant was precipitated with 1-7 (lane c and d) and analysed by SDS-PAGE.

Fig. 9

Expression of Fc$_\epsilon$R on B cells from normal, non-atopic individuals. PBL were stained with FITC-anti BI and biotin-8-30 followed by TR-avidin. A representative result of the two color FACS analysis is shown in panel A and the histograms of Fc$_\epsilon$R positive cells in B$^+$ cells of several donors are shown in panel B.

Fig. 10

Expression of Fc$_\epsilon$R on B cells from atopic patients. As described in Fig. 9 the two color FACS analysis is shown in panel A, and the histograms of Fc$_\epsilon$R positive cells in B$_1$+ cells from several patients are shown in panel B.

## Fig. 11

Induction of $Fc_\epsilon R$ in Fc R⁺B cell population (panel A), but not in $Fc_\epsilon R$⁻B cell population (panel B). Tonsillar $Fc_\epsilon R$⁺ and $Fc_\epsilon R$⁻B cells were cultured for 48 hr with or without PHA-sup in the presence or absence of 10 μg/ml IgE or IgG, and analyzed for the expression of $Fc_\epsilon R$ by FACS.

## Fig. 12

Failure to induce $Fc_\epsilon R$ expression in T cells in the mixed lymphocyte reactions in the presence of IgE. Allogeneic lymphocytes ($1 \times 10^6$ /ml) were cultured with 10 μg/ml IgE for 4 days, and the expression of $Fc_\epsilon R$ on Bl-positive cells and Lue 4-positive cells was analyzed by two-color FACS.

## Fig. 13

Expressions of $Fc_\epsilon R$ and $Fc_\gamma R$ on tonsillar MNC. Tonsillar MNC were doubly stained with (A) biotinated anti-$Fc_\epsilon R$ and FITC-anti-Bl, FITC-anti-IgD, PC-anti-IgM, FITC-anti-IgA or PC-anti-IgG, (B) biotinated human IgE and FITC-anti-Bl, FITC-anti-IgD, PC-anti-IgM, FITC-anti-IgA and PC-anti-IgG, (C) biotinated human IgGl and FITC-anti-Bl, FITC-anti-IgD, PC-anti-IgM, FITC-anti-IgA. Biotinated reagents were developed with TR-avidin for double staining with FITC antibodies or FITC-avidin for double staining with PC-antibodies.

## Fig. 14

$Fc_\epsilon R$ expression on various isotype positive B cells from normal PBL. PBL from a normal individual were doubly stained with biotinated anti-$Fc_\epsilon R$ and FITC-anti-IgD, PC-anti-IgM, FITC-anti-IgA or PC-anti-IgG. The fluorescence intensities of $Fc_\epsilon R$ staining on IgD⁺(A), IgM⁺(B), IgA⁺(C) or IgG⁺(D) B cells are shown on the histograms.

## Fig. 15

$Fc_\epsilon R$ expression on various isotype positive cells from PBL of patients with hyper IgE-syndrome. PBL from two hyper IgE patients were doubly stained with biotinated anti-$Fc_\epsilon R$ and FITC-anti-IgD, PC-anti-IgM, PC-anti-IgG or FITC-anti-IgA or PC-anti-$Fc_\epsilon R$ and Biotinated anti-IgE. Biotinated antibodies were developed with TR-avidin or FITC avidin. The fluorescence intensities of Fc R staining on IgD⁺(A), IgM⁺(B), IgG⁺(C), IgA⁺(D); or IgE⁺(E) B cells are shown on the histograms. Solid and dotted line represent the histograms of PBL from each of two patients.

## Fig. 16

Induction of $Fc_\epsilon R$ on bone marrow B cells. Bone marrow MNC from an immunologically normal adult donor were incubated without (-) or with IgE (10μg/ml), PHA-sup (10 % v/v), PHA-sup plus IgG (10 μg/ml) or PHA-sup plus IgE for 3 days. After culture, cells were doubly stained with biotinated anti-$Fc_\epsilon R$ and FITC-anti-Bl or FITC-anti-Ig and developed with TR-avidin. Cells were analyzed by FACS. The intensities of $Fc_\epsilon R$ staining on Bl + B cells (a) and IgD⁺ B cells (B) were shown in the histogram.

## Fig. 17

$Fc_\epsilon R$ can be induced on δ⁺ cells but no δ⁻ cells in tonsils. Tonsillar MNC were stained with anti-$Fc_\epsilon R$ and anti-IgD and δ⁻/$Fc_\epsilon R$⁻(A) and δ⁺/$Fc_\epsilon R$⁻(B) cells were sorted by FACS. Sorted cells were indubated with (—) or without (— — — —) PHA-sup (10 % v/v) and IgE (10 μg/ml) for 3 days and stained with anti-$Fc_\epsilon R$ and anti-Bl. The intensities of $Fc_\epsilon R$ staining of Bl + B cells were shown in the histograms.

Fig. 18

Fc$_\epsilon$R is inducible on Fc$_\epsilon$R negative PBL from a patient with CVI. PBL from a patient with CVI were incubated without (A) or with PHA-sup (10 % v/v) (B) or PHA-sup and IgE (10 $\mu$g/ml) (C) for 3 days. After incubation, cells were stained with anti-Fc $_\epsilon$R and anti-BI and analyzed by FACS. The intensities of Fc $_\epsilon$R staining of BI positive B cells are shown in the histograms.

Fig. 19

A sheme for the correlation of the Fc$_\epsilon$R expression and human B cell differentiation.

**Claims**

1. Monoclonal antibodies recognizing surface receptors for IgE (Fc$_\epsilon$R) on human B lymphocytes.

2. Monoclonal antibodies as claimed in claim 1 wherein expressed Fc$_\epsilon$R on 8866, 1788, CESS and SKW-C14 cells are recognized.

3. Monoclonal antibodies as claimed in claims 1 and 2 wherein these antibodies recognize a major component of the receptor having a molecular weight of about 46Kd and a minor component of the receptor having a molecular weight of about 24 to 29Kd.

4. Monoclonal antibody designated as (3-5) as claimed in claim 3 wherein this antibody is of type $\gamma_1$ and does not significantly inhibit the binding of IgE.

5. Monoclonal antibody designated as (8-30) as claimed in claim 3 wherein this antibody is of type $\mu$ and inhibits the binding of IgE.

6. Monoclonal antibody designated as (1-7) as claimed in claim 3 wherein this antibody is of type $\alpha_{2b}$ and inhibits the binding of IgE.

7. Monoclonal antibody as claimed in any of the claims 3 to 6, wherein this antibody is capable precipitating a major band of about 46Kd and a minor band of about 24 to 29Kd from a lysate of Fc$_\epsilon$R-positive 8866 cells.

8. Monoclonal antibody as claimed in claims 4, 6 and 7, wherein these antibodies bind to two different specific epitopes of Fc$_\epsilon$R.

9. Monoclonal antibody as claimed in claim 5, wherein this antibody binds to a specific epitope of Fc$_\epsilon$R different from the specific epitope to which the monoclonal antibodies as claimed in the claims 4, 6 or 7 bind.

10. Monoclonal antibodies as claimed in any of the claims 1 to 9, which are detachably labeled.

11. Monocolonal antibodies as claimed in claim 10, wherein the detectable label is a radioactive isotope, an enzyme, a fluorescent or chemiluminescent compound.

12. A kit useful for the detection of B cells expressing surface receptor for IgE comprising

(a) a solid phase carrying a monoclonal antibody specific for said surface receptor for IgE as claimed in any of the claims 1 to 9; and

(b) a monoclonal antibody to said surface receptor for IgE bound to a detectable label.

13. An immunoassay method for the determination of B cells expressing surface receptor for IgE which comprises

(a) incubating a sample containing or suspected of containing said surface receptor for IgE with a monoclonal antibody to said surface receptor for IgE as claimed in any of the claims 1 to 9;

(b) determining the amount of said monoclonal antibody bound to said surface receptor for IgE or remaining unbound.

14. An immunoassay method as claimed in claim 13, wherein a biotinated monoclonal antibody is used as monoclonal antibody, followed by Texas-Red avidin and by dual laser FACS analysis.

15. An immunoassay method for the determination of IgE receptors in biological materials which comprises

(a) incubating a sample containing or suspected of containing said receptor for IgE with a monoclonal antibody to said surface or soluble receptor for IgE as claimed in any of the claims 1 to 9, and

(b) determining the amount of said receptor for IgE.

16. An immunoassay method as claimed in claim 15, wherein blood or serum is used as biological material.

17. Process for preparing new monoclonal antibodies as claimed in any of the claims 1 to 9, wherein the hybrid cell line producing the antibodies is prepared by cell fusion and subcloned and after growth of the cells the monoclonal antibodies with anti-Fc$_\epsilon$R-specifity are isolated and, if desired, subsequently the obtained antibodies are detectable labeled.

18. Process as claimed in claim 17, wherein spleen cells from Balb/c mice cells immunised with B lymphoblastoid cells secreting Fc$_\epsilon$R are used for cell fusion.

19. Process as claimed in claim 18, wherein 8866 cells are used as B lymphloblastoid cells secreting Fc$_\epsilon$R.

20. Process as claimed in claim 17, wherein P3UI mouse myeloma cells are used as myeloma cells.

21. Process as claimed in claims 17 to 19, wherein the selected monoclonal clones secreting anti-Fc$_\epsilon$R antibody activity are transplanted in Balb/c mice and the formed ascitic fluid is isolated and, if desired, the obtained monoclonal antibody is subsequently detectable labeled.

22. Process as claimed in claims 17 or 21, wherein said detectable label is a radioactive isotope, an enzyme, a fluorescent or a chemiluminescent compound.

23. A hybridoma capable of secreting a monoclonal antibody with anti-Fc$_\epsilon$R specifity as claimed in any of the claims 1 to 9.

24. A hybridoma as claimed in claim 23, wherein said hybridoma is obtained by cell fusion of spleen cells of a mouse immunised with lymphoblastoid B cells secreting Fc$_\epsilon$R and myeloma cells.

25. A hybridoma as claimed in claims 23 or 24, wherein said hybridoma permanently produces the monoclonal antibody as claimed in claims 4, 7 and 8.

26. A hybridoma as claimed in claims 23 or 24, wherein said hybridoma permanently produces the monoclonal antibody as claimed in claims 5 and 9.

27. A hybridoma as claimed in claim 23 or 24, wherein said hybridoma permanently produces the monoclonal antibody as claimed in claims 6, 7 and 8.

28. A hybridoma as claimed in claim 25, deposited at the C.N.C.M under the file number I-583.

29. A hybridoma as claimed in claim 26, deposited at the C.N.C.M under the file number I-584.

30. Process for preparing a hybridoma as claimed in any of the claims 23 to 29, wherein said hybridoma is obtained by cell fusion of spleen cells of a mouse immunised with lymphoblastoid B cells secreting Fc$_\epsilon$R and myeloma cells.

31. Process as claimed in claim 30, wherein said myeloma cells are those of the line P3UI.

Fig. 1

Fig. 2

Fig. 3

Fig. 4

0 254 767

Fluorescence Intensity

Fig. 5

0 254 767

Fig. 6

Fig. 7

Fig. 8

Fig. 9

0 254 767

Fig. 10

Fig. 11

Fig. 12

fl/Leu4

fl/B1

bi/8-30

Fig. 13

Fig. 14

Fluorescence Intensity

Fluorescence Intensity

Fig. 15

Fig. 16

Fig. 17

Fig. 18

0 254 767

Bone Marrow    PBL and Tonsils

$IgM^+$  
$Fc\varepsilon\text{-}R^-$

$IgM^+IgD^+$  
$Fc\varepsilon\text{-}R^-$  
(inducible)

$IgM^+IgD^+$  
$Fc\varepsilon\text{-}R^+$

$IgG^+, IgA^+$ or $IgE^+$  
$Fc\varepsilon\text{-}R^-$  
(not inducible)

Fig. 19

0 254 767

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 106, no. 1, January 1987, page 351, abstract no. 3603s, Columbus, Ohio, US; H. KIKUTANI et al.: "Fcepsilon receptor, a specific differentiation marker transiently expressed on mature B cells before isotype switching", & J. EXP. MED. 1986, 164(5), 1455-69 * Whole abstract * | 1,2,10 -16,22 ,23 | C 12 P 21/00<br>G 01 N 33/577<br>C 12 N 5/00<br>C 12 N 15/00 //<br>C 07 K 15/00 |
| | --- | | |
| | CHEMICAL ABSTRACTS, vol. 103, no. 11, September 1985, page 162, abstract no. 86103r, Columbus, Ohio, US; E. RECTOR et al.: "Detection and characterization of monoclonal antibodies specific to IgE receptors on human lymphocytes by flow cytometry", & IMMUNOLOGY 1985, 55(3), 481-8 * Whole abstract * | 1,2,5, 6,10- 24,30 | |
| | | | **TECHNICAL FIELDS SEARCHED (Int. Cl.4)** |
| | --- | | C 12 P |
| X | CHEMICAL ABSTRACTS, vol. 104, no. 15, 14th April 1986, page 556, abstract no. 128114y, Columbus, Ohio, US; & JP-A-60 255 734 (NICHIREI CORP.) 17-12-1985 * Whole abstract * | 1,2,5, 6,10- 24,30 | G 01 N<br>A 61 K<br>C 12 N |
| | ---     -/- | | |

The present search report has been drawn up for all claims

| Place of search | Date of completion of the search | Examiner |
|---|---|---|
| THE HAGUE | 03-04-1987 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 105, no. 7, 18th August 1986, page 490, abstract no. 59181z, Columbus, Ohio, US; M. CAPRON et al: "Functional study of a monoclonal antibody to IgE Fc receptor (FceR2) of eosinophils, platelets, and macrophages", & J. EXP. MED. 1986, 164(1), 72-89 * Whole abstract * | 1-3,5-7,10-16,22,23 | |
| | --- | | |
| X | CHEMICAL ABSTRACTS, vol. 105, no. 12, September 1986, page 487, abstract no. 95741r, Columbus, Ohio, US; T. NAKAJIMA et al.: "IgE receptors on human lymphocytes. I. Identification of the molecules binding to monoclonal anti-Fce receptor antibodies", & EUR. J. IMMUNOL. 1986, 16(7), 809-14 * Whole abstract * | 1-3,5-7,10-16,22,23 | |
| | --- | | |
| X | BIOLOGICAL ABSTRACTS, vol. 80, no. 11, November 1985, abstract no. 95522, Biological Abstracts Inc., Philadelphia, US; E. RECTOR et al.: "Detection and characterization of monoclonal antibodies specific to immunoglobulin E receptors on human lymphocytes by flow cytometry", & IMMUNOLOGY, 55(3), 481-488, 1985 * Whole abstract * | 1,2,5,6,10-24,30 | |
| | ---     -/- | | **TECHNICAL FIELDS SEARCHED (Int. Cl.4)** |

The present search report has been drawn up for all claims

| Place of search | Date of completion of the search | Examiner |
|---|---|---|
| THE HAGUE | 03-04-1987 | OSBORNE H.H. |

## DOCUMENTS CONSIDERED TO BE RELEVANT

Page 3

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 104, no. 3, January 1986, page 379, abstract 18371x, Columbus, Ohio, US; S.A. HUDAK et al.: "A microtiter plate assay for detecting IgE-binding molecules and cells bearing Fc receptors for IgE", & J. IMMUNOL. METHODS 1985, 84(1-2), 11-24 * Whole abstract * | 12,13, 15,16 | |

-----

TECHNICAL FIELDS SEARCHED (Int. Cl.4)

The present search report has been drawn up for all claims

| Place of search | Date of completion of the search | Examiner |
|---|---|---|
| THE HAGUE | 03-04-1987 | OSBORNE H.H. |